(19) Europäisches Patentamt
European Patent Office
Office européen des brevets

(11) Veröffentlichungsnummer: **0 464 539 A2**

# (12) EUROPÄISCHE PATENTANMELDUNG

(21) Anmeldenummer: **91110323.2**

(22) Anmeldetag: **22.06.91**

(51) Int. Cl.5: **C07C 211/15**, C07C 209/62

(30) Priorität: **06.07.90 DE 4021605**

(43) Veröffentlichungstag der Anmeldung: **08.01.92 Patentblatt 92/02**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Böhm, Stefan, Dr.**
**Carl-Leverkus-Strasse 30**
**W-5090 Leverkusen(DE)**
Erfinder: **Marhold, Albrecht, Dr.**
**Carl-Duisberg-Strasse 329**
**W-5090 Leverkusen(DE)**
Erfinder: **Beck, Gunther, Dr.**
**Am Mittelberg 19**
**W-5090 Leverkusen(DE)**

## (54) Verfahren zur Herstellung von beta-Fluor-tert.-alkylaminhydrochloriden und beta-Fluor-tert.-alkylamiden.

(57) Verfahren zur Herstellung von β-Fluor-tert.-alkylamin-Hydrochloriden der Formel (I)

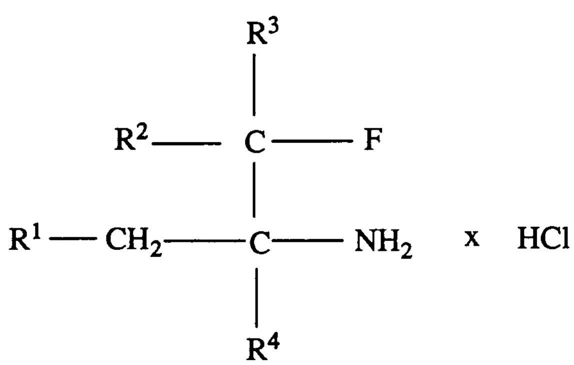

(I)

dadurch gekennzeichnet, daß man Fluorallyl-Derivate der Formel (IIa)

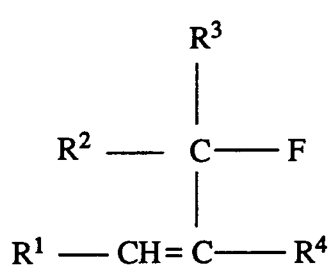

(IIa)

EP 0 464 539 A2

oder β-Fluor-tert.-alkylalkohole der Formel (IIb)

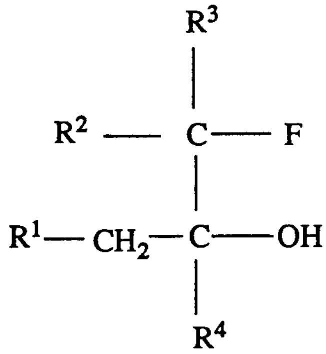

(IIb)

mit Nitrilen der Formel (III)

$R^5$ - CN (III)

in Gegenwart von Schwefelsäure und gegebenenfalls in Gegenwart eines Verdünnungsmittels umsetzt zu den β-Fluor-tert.-alkylamiden der Formel (IV)

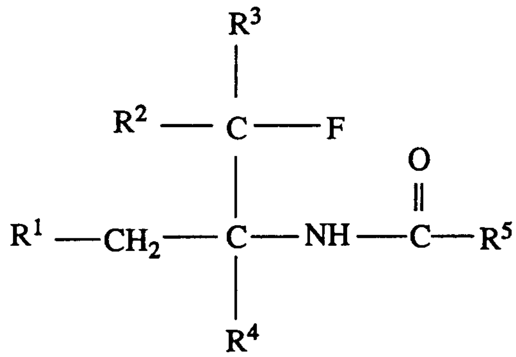

(IV)

und diese gegebenenfalls verseift.

Die Erfindung betrifft ein neues Verfahren zur Herstellung von β-Fluor-tert.-alkylamin-hydrochloriden und β-Fluor-tert.-alkylamiden, die als Zwischenprodukte zur Herstellung von Verbindungen mit herbizider und insektizider Wirksamkeit verwendet werden können.

Es ist bereits bekannt, daß man Fluor-tert.-alkylamine erhält, wenn man Chlortert.-alkylcarbonsäurechlorid mit Kaliumfluorid zu Fluor-tert.alkylcarbonsäurefluorid umsetzt und dieses mit Trimethylsilylazid in das Fluor-tert.-alkylisocyanat überführt, das dann anschließend zum Amin verseift wird (vgl. Synthesis, 1972, 551-553, DE-OS 3 611 195). Nachteilhaft an diesem Verfahren ist der hohe Preis der Ausgangsprodukte, insbesondere von Chlor-tert.-pivaloylchlorid, und die Verwendung des teuren und sicherheitstechnisch problematischen Trimethylsilylazids.

Es ist weiterhin bekannt, daß durch Umsetzung von Nitrilen mit bestimmten Halogenalkenen oder Halogenalkoholen bestimmte N-(2-Halogen-1-ethyl)-amide hergestellt werden können (vgl. R.M. Lusskinn und JJ. Ritter, J.Am. Chem Soc. 72, 5577-5578 (1950)). So wird lediglich die Herstellung von N-(2-Chlor- bzw. Brom-1-ethyl)-amiden beschrieben, während das vorliegende Verfahren die Herstellung von Fluor-tert.-alkylamiden beschreibt. Nachteilhaft an dem in o.g. J. Am. Chem. Soc. beschriebenen Verfahren zur Herstellung von N-(2-Chlor- bzw. -Brom-1-ethyl)-amiden sind die schlechten Ausbeuten.

Es wurde nun gefunden, daß man β-Fluor-tert.-alkylamine der Formel (I) in Form ihrer Hydrochloride

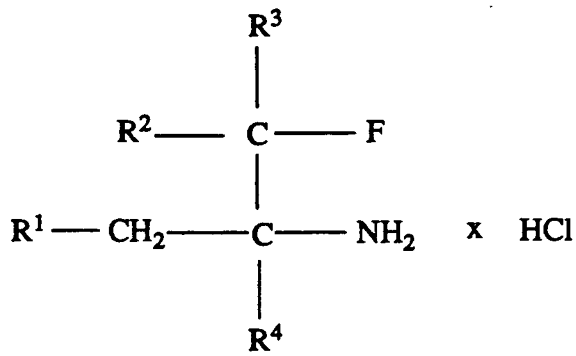

(I)

in welcher

$R^1$, $R^2$ und $R^3$ unabhängig voneinander für Wasserstoff, Alkyl, Halogenalkyl oder Alkoxyalkyl stehen und

$R^4$ für Alkyl, Halogenalkyl, Alkoxylalkyl oder gegebenenfalls substituiertes Aryl steht,

erhält, wenn man Fluorallyl-Derivate der Formel (IIa)

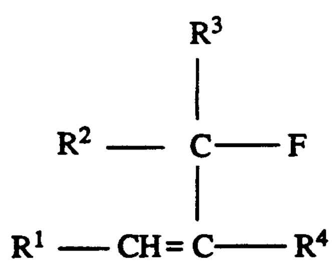

(IIa)

oder β-Fluor-tert.-alkylkohole der Formel (IIb)

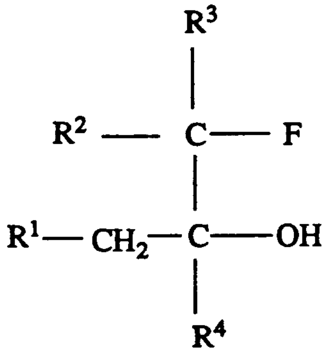

(IIb)

wobei
$R^1$, $R^2$, $R^3$ und $R^4$ die oben angegebene Bedeutung haben,
mit Nitrilen der Formel (III)

$R^5$-CN (III)

in welcher

$R^5$ für Wasserstoff, Alkyl, Halogenalkyl oder jeweils gegebenenfalls substituiertes Aryl oder Phenylalkyl steht,

in Gegenwart von Schwefelsäure und gegebenenfalls in Gegenwart eines Verdünnungsmittels umsetzt zu den $\beta$-Fluor-tert.-alkylamiden der Formel (IV)

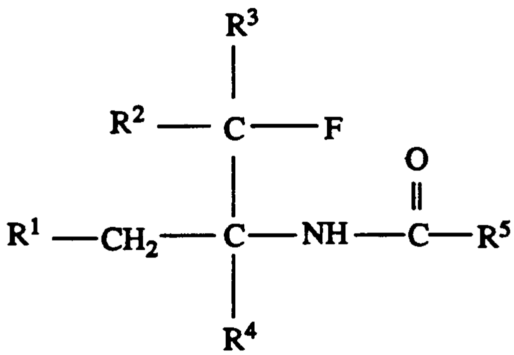

(IV)

in welcher $R^1$, $R^2$, $R^3$, $R^4$ und $R^5$ die oben angegebene Bedeutung haben,
und diese dann gegebenenfalls nach Zwischenisolierung verseift.

Es ist als überraschend zu bezeichnen, daß Fluorallyl-Derivate der Formel (IIa) und $\beta$-Fluor-tert-alkylkohole der Formel (IIb) unter den Reaktionsbedingungen in so guten Ausbeuten zu den gewünschten Produkten reagiert, da von analogen Chlorverbindungen keine vergleichbar guten Ausbeuten in der Literatur berichtet werden. Außerdem ist es als überraschend zu bezeichnen, daß die Verseifung der Formamide zu den Fluor-tert.-alkylaminhydrochloriden in so hohen Ausbeuten gelingt, da von N-(2-haloalkyl)-amiden bekannt ist, daß sie sehr leicht, zum Teil bereits bei Raumtemperatur, einer cyclisierenden Umwandlung in Oxazoline unter Abspaltung von Halogenwasserstoff unterliegen. Außerdem ist bekannt, daß N-(2-haloalkyl)-amide sehr leicht unter Verlust des Halogenatoms zu Hydroxy-Verbindungen verseift werden (siehe z.B. R.M. Lusskin und J.J. Ritter, J. Am. Chem. Soc. 72, 5577-5578 (1950) und T.L. Cairns et al., J. Org. Chem. 17, 751-757(1952)).

Das erfindungsgemäße Verfahren zeichnet sich durch eine Reihe von Vorteilen gegenüber dem Stand der Technik aus. So ermöglicht es die Herstellung von Fluor-tert.-alkylaminen bzw. von $\beta$-Fluor-tert.-alkylamiden in hohen Ausbeuten aus einfachen Grundchemikalien und ist einfach durchzuführen. Insbesondere werden bei dem erfindungsgemäßen Verfahren keine teuren Agenzien benötigt.

Die Kohlenstoffketten in Alkyl, Alkoxyalkyl, Halogenalkyl oder im Alkylteil von Phenylalkyl sind jeweils geradkettig oder verzweigt.

Halogen steht für Fluor, Chlor, Brom oder Iod. Vorzugsweise steht Halogen für Fluor, Chlor oder Brom, insbesondere für Fluor oder Chlor.

Alkyl steht vorzugsweise für Alkyl mit 1 bis 6 Kohlenstoffatomen. Besonders bevorzugt steht Alkyl für Methyl, Ethyl, n-Propyl, iso-Propyl, n-, iso-, sec.- oder tert.-Butyl, insbesondere für Methyl oder Ethyl.

Halogenalkyl steht vorzugsweise für Halogenalkyl mit 1 bis 6, insbesondere mit 1 bis 4, Kohlenstoffatomen. Besonders bevorzugt seien Halogenmethyl und Halogenethyl mit jeweils 1, 2 oder 3 Fluor- und/oder Chloratomen genannt.

Alkoxyalkyl steht vorzugsweise für Alkoxyalkyl mit 1 bis 4, insbesondere 1 oder 2, Kohlenstoffatomen im jeweiligen Alkylteil. Insbesondere seien Methoxymethyl, Ethoxymethyl, Methoxyethyl und Ethoxyethyl genannt.

Aryl in $R^4$ steht vorzugsweise für Phenyl oder Naphthyl, insbesondere für Phenyl.

Aryl in $R^5$ steht vorzugsweise für Phenyl.

Die Arylsubstituenten sind vorzugsweise ausgewählt aus der Gruppe Halogen, Alkyl mit 1 bis 4, insbesondere 1 bis 3, Kohlenstoffatomen, Halogenalkyl mit 1 bis 4, insbesondere 1 oder 2, Kohlenstoffatomen, oder Alkoxy mit 1 bis 4, insbesondere 1 oder 2, Kohlenstoffatomen.

Besonders bevorzugt sind die Arylsubstituenten ausgewählt aus der Gruppe Fluor, Chlor, Brom, Methyl, Ethyl, n- oder iso-Propyl, Trifluormethyl, Trichlormethyl, Methoxy und Ethoxy.

Phenylalkyl steht vorzugsweise für Phenylalkyl mit 1 bis 4 Kohlenstoffatomen, insbesondere für Benzyl oder Phenylethyl.

Die Phenylsubstituenten sind vorzugsweise bzw. besonders bevorzugt ausgewählt aus der Gruppe, die bereits oben für die Arylsubstituenten angegeben ist.

In den allgemeinen Formeln stehen $R^1$, $R^2$ und $R^3$ unabhängig voneinander vorzugsweise für Wasserstoff, Alkyl mit 1 bis 6, insbesondere 1 bis 4, Kohlenstoffatomen, Halogenalkyl mit 1 bis 6, insbesondere 1 bis 4, Kohlenstoffatomen oder Alkoxyalkyl mit jeweils 1 bis 4 Kohlenstoffatomen in den Alkylteilen.

$R^1$, $R^2$ und $R^3$ stehen unabhängig voneinander besonders bevorzugt für Wasserstoff, Methyl, Ethyl, n- oder iso-Propyl, n-, iso- oder sec.-Butyl, Halogenmethyl oder Halogenethyl mit 1, 2 oder 3 Fluor- und/oder Chloratomen, Methoxymethyl, Ethoxymethyl, Methoxyethyl oder Ethoxyethyl. $R^1$, $R^2$ und $R^3$ stehen unabhängig voneinander insbesondere für Wasserstoff, Methyl oder Ethyl. Besonders hervorzuheben ist die Bedeutung von Wasserstoff für $R^1$, $R^2$ und $R^3$.

In den allgemeinen Formeln steht $R^4$ vorzugsweise für Alkyl oder Halogenalkyl mit jeweils 1 bis 6, insbesondere 1 bis 4, Kohlenstoffatomen, Alkoxyalkyl mit jeweils 1 bis 4 Kohlenstoffatomen im Alkylteil oder für jeweils unsubstituiertes oder einfach bis fünffach, insbesondere einfach bis dreifach gleich oder verschieden substituiertes Phenyl oder Naphthyl, wobei die Substituenten ausgewählt sind aus der Gruppe Halogen, $C_1$-$C_4$-Alkyl, Halogen-$C_1$-$C_4$-alkyl und $C_1$-$C_4$-Alkoxy.

$R^4$ steht besonders bevorzugt für Methyl, Ethyl, n- oder i-Propyl oder Halogenmethyl oder Halogenethyl mit 1, 2 oder 3 Fluor- oder Chloratomen, Methoxymethyl, Ethoxymethyl, Methoxyethyl, Ethoxyethyl oder für unsubstituiertes oder einfach oder zweifach, gleich oder verschieden substituiertes Phenyl, wobei die Substituenten ausgewählt sind aus der Gruppe Fluor, Chlor, Methyl, Ethyl, n- oder iso-Propyl, Halogen-$C_1$-$C_2$-alkyl, insbesondere Trifluormethyl und Trichlormethyl, Methoxy und Ethoxy.

Besonders hervorzuheben ist die Bedeutung von Methyl und Ethyl für $R^4$.

$R^5$ steht in den allgemeinen Formeln vorzugsweise für Wasserstoff, Alkyl mit 1 bis 6, insbesondere 1 bis 4, Kohlenstoffatomen, Halogenaklyl mit 1 bis 6, insbesondere 1 bis 4, Kohlenstoffatomen oder für jeweils unsubstituiertes oder im Phenylteil einfach bis fünffach, insbesondere einfach bis dreifach, gleich oder verschieden substituiertes Phenyl oder Phenyl-$C_1$-$C_4$-alkyl, wobei als Substituenten die bereits oben bei $R^4$ genannten Phenylsubstituenten ausgewählt sind.

$R^5$ steht besonders bevorzugt für Wasserstoff, Methyl, Ethyl, Halogen-$C_1$-$C_2$-alkyl,insbesondere Trifluormethyl und Trichlormethyl, Phenyl, Benzyl oder Phenethyl.

Besonders hervorzuheben ist die Bedeutung von Wasserstoff oder Trichlormethyl für $R^5$.

Das erfindungsgemäße Verfahren läßt sich beispielsweise durch folgendes Formelschema darstellen:

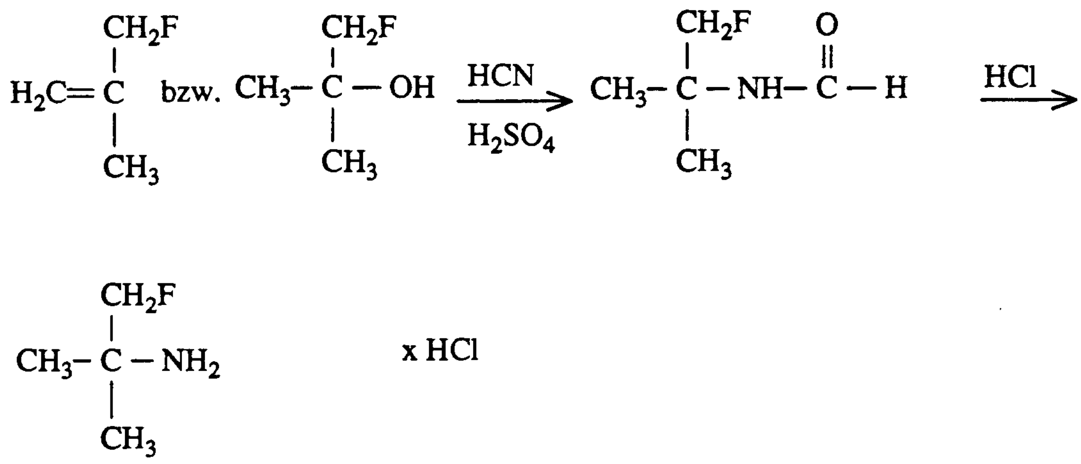


Die Fluorallyl-Derivate der Formel (IIa) bzw. β-Fluor-tert.-alkylalkohole der Formel (IIb) sind bekannt bzw. können nach bekannten Verfahren hergestellt werden (vgl. z.B. J. Mol. Struct. 114, 385-90 (1984); DE-OS 3 608 380, J. Chem. Soc. 1958, 2259; Bull. Soc. Chim. Fr. 1969, 271 oder J. Org. Chem. 53, 1026 (1980)).

Die Nitrile der Formel (III) sind bekannte Verbindungen.

Das erfindungsgemäße Verfahren kann in Gegenwart eines Verdünnungsmittels durchgeführt werden. Hierzu gehören insbesondere Ether, wie Diethylether, Dioxan, Di-n-butylether, Tetrahydrofuran, Ethylenglykoldimethyl- oder -diethylether, oder aliphatische Carbonsäruen wie Essigsärue, Propionsäue, Trifluoressigsäure. Besonders bevorzugt verwendet man überschüssiges Nitril als Lösungsmittel.

Die Reaktionstemperatur kann bei dem erfindungsgemäßen Verfahren in einem größeren Bereich variiert werden im allgemeinen arbeitet man bei der Umsetzung von Verbindungen der Formel (IIa) bzw. (IIb) mit Nitrilen der Formel (III) bei Temperaturen von 0 bis 100° C, vorzugsweise bei 10 bis 40° C, insbesondere bei 20° C bis 40° C.

Das erfindungsgemäße Verfahren wird vorzugsweise bei Normaldruck durchgeführt. Es kann aber auch bei Drucken zwischen 0,1 und 10 bar durchgeführt werden.

Zur Durchführung des erfindungsgemäßen Verfahrens setzt man pro Mol Fluorallyl-Derivat der Formel (IIa) bzw. β-Fluor-tert.-alkylalkohol der Formel (IIb) im allgemeinen 1 bis 5 Mol, vorzugsweise 1 bis 2 Mol des Nitrils der Formel (III) sowie 1 bis 3 Mol, vorzugsweise 1 bis 1,5 Mol Schwefelsäure ein.

Die Reaktion wird so durchgeführt, daß man eine Mischung aus dem Nitril und der Schwefelsäure, gegebenenfalls in Gegenwart eines Verdünnungsmittels vorlegt, und das Fluor-allyl-Derivat bzw. den β-Fluor-tert.-alkylalkohol, gegebenenfalls im Gemisch mit dem Nitril oder einem Verdünnungsmittel zutropft, und bis zur vollständigen Umsetzung rührt. Die Isolierung der Fluor-tert.-alkylamide der Formel (IV) erfolgt in üblicher Weise dadurch, daß man beispielsweise das Reaktionsgemisch auf Eis gibt, gegebenenfalls neutralisiert und das Produkt mit einem wasserunlöslichen organischen Lösungsmittel extrahiert, mit Wasser wäscht, trocknet und das organische Lösungsmittel destillativ entfernt.

Die anschließende Verseifung der Verbindungen der Formel (IV) wird vorzugsweise mit wäßriger Salzsäure durchgeführt. Man kann sie auch als "Eintopfreaktion" ohne vorherige Isolierung des Amids der Formel (IV) ausführen.

Besonders bevorzugt lassen sich diejenigen Verbindungen der Formel (IV) verseifen, in denen $R^5$ für Wasserstoff steht.

Die Verseifung wird im allgemeinen bei Temperaturen von 10 bis 120° C, vorzugsweise bei 40 bis 80° C, durchgeführt.

Die freien Amine erhält man aus den Hydrochlorid-Salzen der Formel (I) durch Neturalisation mit Basen wie Alkalihydroxiden, insbesondere Natriumhydroxid, und anschließende Extraktion und/oder Destillation in bekannter Weise.

Das erfindungsgemäße Verfahren wird durch die folgenden Beispiele veranschaulicht.

Beispiel 1

Verbindung IV-1: N-(Fluor-tert.-butyl)-formamid

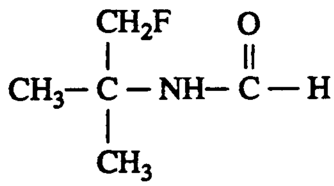

175 g (1,5 Mol) 85 %ige Schwefelsäure und 40 ml (1 Mol) Blausäure werden bei Raumtemperatur vorgelegt. Dazu tropft man unter Rühren bei Raumtemperatur eine Mischung von 75 g (1 Mol) Methallylfluorid und 40 ml (1 Mol) Blausäure. Dann wird 1 Stunde bei 40° C Badtemperatur und 15 Stunden bei Raumtemperatur nachgerührt. Überschüssige Blausäure wird im Vakuum abgezogen, der Rückstand auf 750 ml Eiswasser gegossen und 5 mal mit 200 ml Methylenchlorid extrahiert Nach Trocknen mit $MgSO_4$ wird das Lösungsmittel im Vakuum abgezogen.

| | |
|---|---|
| Ausbeute: | 101,4g (85 % der Theorie) |
| Siedepunkt: | 53-55° C (0,05 mbar) |
| Reinheit: | 97 % (Gaschromatographie) |

Verbindung IV-1: N-(Fluor-tert.-butyl)-formamid
alternative Herstellung:

92 g (1 Mol) 1-Fluor-2-methyl-propan-2-ol werden bei 10 bis 15° C in 80 ml (2 Mol) Blausäure gelöst. Unter Rühren werden langsam 140 g (1,4 Mol) konz. Schwefelsäure zugetropft Dann wird 15 Stunden bei Raumtemperatur nachgerührt.

Überschüssige Blausäure wird im Vakuum abgezogen, der Rückstand wird auf 250 ml Eiswasser gegeben und 5 mal mit 100 ml Dichlormethan extrahiert. Nach Trocknen mit Magnesiumsulfat wird das Lösungsmittel im Vakuum abdestilliert.

| | |
|---|---|
| Ausbeute: | 86g (72 % der Theorie) |
| Siedepunkt: | 53-54° C (0,05 mbar) |
| Reinheit: | 95 % (Gaschromatographie) |

Verbindung I-1: N-(Fluor-tert.-butyl)-aminhydrochlorid

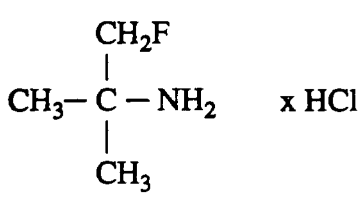

119 g (1 Mol) N-(Fluor-tert.-butyl)-formamid werden mit 1.000 ml 20 %iger Salzsäure versetzt und 4 Stunden bei 70° C gerührt. Anschließend destilliert man unter vermindertem Druck bis zur Trockne und trocknet den Rückstand im Vakuum bis zur Gewichtskonstanz.

| | |
|---|---|
| Ausbeute: | 120 g (94 %der Theorie) |
| Schmelzpunkt: | 250° C |

Beispiel 2

Verbindung IV-2: N-(Fluor-tert.-butyl)-benzamid

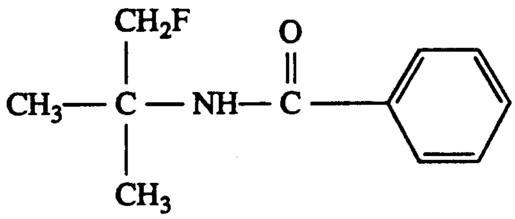

87,5 g (0,75 Mol) 85 %ige Schwefelsäure und 52 g (0,5 Mol) Benzonitril werden bei Raumtemperatur vorgelegt. Dazu tropft man unter Rühren bei Raumtemperatur eine Mischung von 37 g (0,5 Mol) 3-Fluor-2-methyl-propen und 52 g (0,5 Mol) Benzonitril. Die Reaktionswärme wird durch Eiskühlung abgeführt. Nach einer Stunde Nachrühren ist die Reaktion abgeschlossen und das Produkt fällt aus. Man nimmt das Gemisch mit Wasser auf und extrahiert mit Ether. Die organische Phase wird mit $MgSO_4$ getrocknet, das Lösungsmittel abgezogen und der Rückstand im Vakuum bis zur Gewichtskonstanz getrocknet. Nach Waschem mit Hexan erhält man das Produkt in Form farbloser Kristalle.

Ausbeute: 71 g (73 % der Theorie)
Schmelzpunkt: 96-97 °C
Reinheit: 98 % (Gaschromatographie)

Verbindung IV-2: N-(Fluor-tert.-butyl)-benzamid
alternative Herstellung:

73,5 g (0,75 Mol) konz. Schwefelsäure und 52 g (0,5 Mol) Benzonitril werden bei Raumtemperatur vorgelegt: Dazu tropft man unter Rühren bei Raumtemperatur eine Mischung von 37 g (0,5 Mol) 1-Fluor-2-methyl-propan-2-ol und 52 g (0,5 Mol) Benzonitril. Die Reaktionswärme wird durch Eiskühlung abgeführt. Nach einer Stunde Nachrühren ist die Reaktion abgeschlossen und das Produkt fällt aus. Man nimmt das Gemisch mit Wasser auf und extrahiert mit Ether. Die organische Phase wird mit $MgSO_4$ getrocknet, das Lösungsmittel abgezogen und der Rückstand im Vakuum bis zur Gewichtskonstanz getrocknet. Nach Waschen mit Hexan erhält man das Produkt in Form farbloser Kristalle.

Ausbeute: 71 g (73 % der Theorie)
Schmelzpunkt: 96-97 °C
Reinheit: 98 % (Gaschromatographie)

Die nach dem erfindungsgemäßen Verfahren herstellbaren Fluor-tert.-alkylamin-Hydrochloride bzw. Fluor-tert.-alkylamide sind Ausgangsstoffe zur Synthese von biologisch aktiven Verbindungen wie z.B. zur Synthese von substituierten Triazolinonen (vgl. EP-A 294 666), welche gute herbizide Eigenschaften besitzen, oder zur Synthese von insektizid wirksamen N-substituierten Benzamiden (vgl. EP-A 243 668).

## Patentansprüche

**1.** Verfahren zur Herstellung von $\beta$-Fluor-tert.-alkylamin-Hydrochloriden der Formel (I)

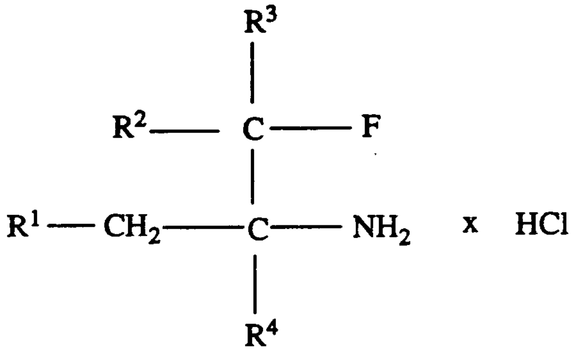

(I)

in welcher

$R^1$, $R^2$ und $R^3$ unabhängig voneinander für Wasserstoff, Alkyl, Halogenalkyl oder Alkoxyalkyl stehen und

$R^4$ für Alkyl, Halogenalkyl, Alkoxyalkyl oder gegebenenfalls substituiertes Aryl steht,

dadurch gekennzeichnet, daß man Fluorallyl-Derivate der Formel (IIa)

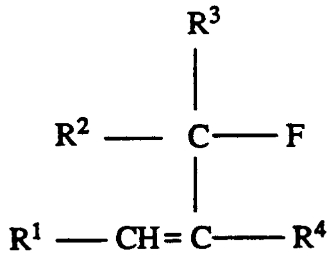

(IIa)

oder β-Fluor-tert.-alkylalkohole der Formel (IIb)

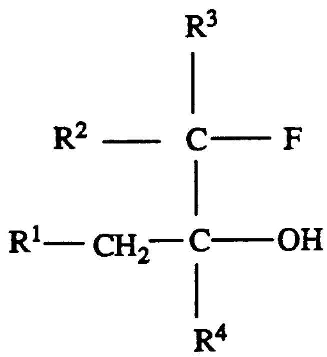

(IIb)

wobei

$R^1$, $R^2$, $R^3$ und $R^4$ die oben angegebene Bedeutung haben,

mit Nitrilen der Formel (III)

$R^5$-CN (III)

in welcher

$R^5$ für Wasserstoff, Alkyl, Halogenalkyl oder jeweils gegebenenfalls substituiertes Aryl oder Phenylalkyl steht,

in Gegenwart von Schwefelsäure und gegebenenfalls in Gegenwart eines Verdünnungsmittels umsetzt zu den β-Fluor-tert.-alkylamiden der Formel (IV)

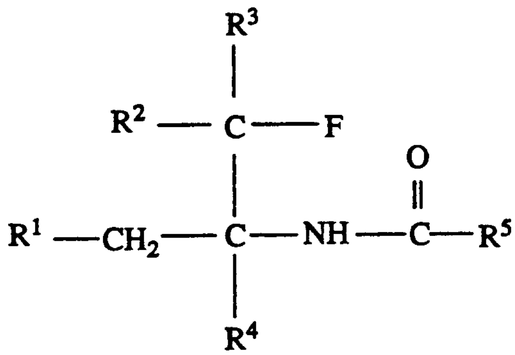

(IV)

in welcher

$R^1$, $R^2$, $R^3$, $R^4$ und $R^5$ die oben angegebene Bedeutung haben,

und diese dann gegebenenfalls nach Zwischenisolierung verseift:

**2.** Verfahren gemäß Anspruch 1, wobei

$R^1$, $R^2$ und $R^3$ unabhängig voneinander für Wasserstoff, Alkyl mit 1 bis 6 Kohlenstoffatomen, Halogenalkyl mit 1 bis 6 Kohlenstoffatomen oder Alkoxyalkyl mit jeweils 1 bis 4 Kohlenstoffatomen in dem Alkylteilen stehen,

$R^4$ für Alkyl oder Halogenalkyl mit jeweils 1 bis 6 Kohlenstoffatomen, Alkoxyalkyl mit jeweils 1 bis 4 Kohlenstoffatomen in den Alkylteilen oder für jeweils unsubstituiertes oder einfach bis fünffach gleich oder verschieden substituiertes Phenyl oder Naphthyl steht, wobei die Substituenten ausgewählt sind aus der Gruppe Halogen, $C_1$-$C_4$-Alkyl, Halogen-$C_1$-$C_4$-alkyl und $C_1$-$C_4$-Alkoxy und

$R^5$ für Wasserstoff, Alkyl oder Halogenalkyl mit jeweils 1 bis 6 Kohlenstoffatomen oder für jeweils unsubstituiertes oder im Phenylteil einfach bis fünffach gleich oder verschieden substituiertes Phenyl oder Phenyl-$C_1$-$C_4$-alkyl steht, wobei als Phenylsubstituenten die bei $R^4$ genannten ausgewählt sind.

**3.** Verfahren gemäß Anspruch 1, dadurch gekennzeichnet, daß man die Umsetzung der Verbindungen (IIa) bzw. (IIb) mit Nitrilen der Formel (III) bei Temperaturen von 0 bis 100 °C durchführt.

**4.** Verfahren gemäß Anspruch 1, dadurch gekennzeichnet, daß man pro Mol Fluor-allyl-Derivat der Formel (IIa) bzw. β-Fluor-tert.-alkylalkohol der Formel (IIb) 1 bis 5 Mol des Nitrils der Formel (III) sowie 1 bis 3 Mol Schwefelsäure einsetzt.

**5.** Verfahren zur Herstellung von β-Fluor-tert.-alkylamiden der Formel (IV)

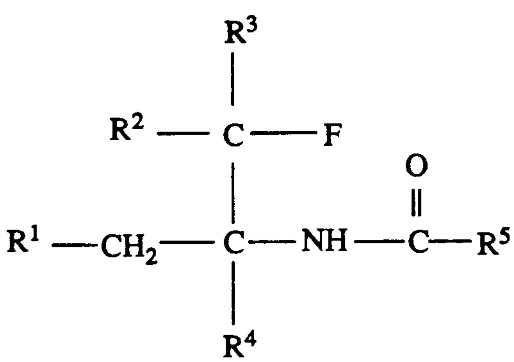

(IV)

in welcher

$R^1$, $R^2$, $R^3$, $R^4$ und $R^5$ die in Anspruch 1 angegebene Bedeutung haben,

dadurch gekennzeichnet, daß man Fluorallyl-Derivate der Formel (IIa)

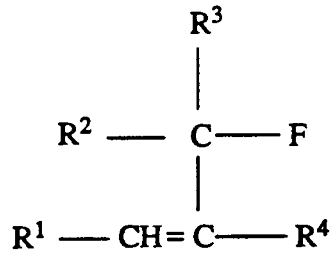

(IIa)

oder β-Fluor-tert.-alkylalkohole der Formel (IIb)

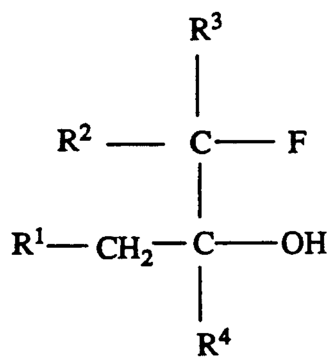

(IIb)

wobei

$R^1$, $R^2$, $R^3$ und $R^4$ die in Anspruch 1 angegebene Bedeutung haben,

mit Nitrilen der Formel (III)

$R^5$-CN (III)

in welcher

$R^5$ die in Anspruch 1 angegebene Bedeutung hat,

in Gegenwart von Schwefelsäure und gegebenenfalls in Gegenwart eines Verdünnungsmittels umsetzt.